# EUROPÄISCHE PATENTSCHRIFT

(11) **EP 2 094 342 B1**
(45) Veröffentlichungstag und Bekanntmachung des Hinweises auf die Patenterteilung: **05.10.2016**
(21) Anmeldenummer: 07846527.5
(22) Anmeldetag: 02.11.2007
(51) Int. Cl.: A61M 25/00, A61M 25/06, A61M 39/02, A61M 39/06, A61M 5/14, F16K 11/02

(54) **KATHETERVORRICHTUNG MIT INFUSIONSPORT UND VENTILEN**
CATHETER APPARATUS WITH INFUSION PORT AND VALVES
DISPOSITIF DE CATHÉTER DOTÉ D'UN PORT DE PERFUSION ET DE SOUPAPES

(30) Priorität: 03.11.2006 US 592595; 30.04.2007 DE 202007006190 U
(43) Veröffentlichungstag der Anmeldung: 02.09.2009
(60) Teilanmeldung: 12198721.8
(73) Patentinhaber: B. Braun Melsungen AG, 34212 Melsungen (DE)
(72) Erfinder: WOEHR, Kevin, 34212 Melsungen (DE)
(74) Vertreter: Kinkeldey, Daniela
(86) Internationale Anmeldenummer: PCT/EP2007/009521
(87) Internationale Veröffentlichungsnummer: WO 2008/052791

(56) Entgegenhaltungen:
- EP-A- 0 268 480
- WO-A-96/40359
- WO-A-99/08742
- US-A- 5 098 405

## Beschreibung

Die Erfindung betrifft eine Kathetervorrichtung nach dem Oberbegriff des Anspruchs 1.

Eine Kathetervorrichtung dieser Art ist aus EP 497 576 B1 bekannt, wobei ein Kombinationsventil im Katheteransatz eingesetzt ist, das einerseits die Portöffnung verschließt und andererseits das Austreten von Blut oder Injektionsflüssigkeit aus dem Katheteransatz in proximale Richtung verhindert. An beiden Ventilbereichen kann Injektionsflüssigkeit eingeführt werden, indem die jeweiligen Ventilbereiche verformt werden.

Weitere Kathetervorrichtung mit Portöffnungen und Ventilelementen sind zum Beispiel aus EP 0 268 480, EP 1 197 242, WO96/40359, US 5 098 405, WO2007/050553 oder US 3 416 567 bekannt.

Der Erfindung liegt die Aufgabe zugrunde, eine solche Kathetervorrichtung mit Port so auszubilden, dass auch eine Flüssigkeitsentnahme, insbesondere Blutentnahme möglich ist.

Diese Aufgabe wird erfindungsgemäß durch den Anspruch 1 gelöst.

Dadurch, dass das zweite Ventilelement als Zwei-Wege-Ventil derart ausgebildet ist, dass eine Durchströmung in beiden Richtungen möglich ist, kann bei Bedarf Flüssigkeit bzw. Blut am proximalen Ende des Katheteransatzes entnommen werden.

Die Erfindung wird beispielsweise anhand der Zeichnung näher erläutert. Es zeigen
- Fig. 1: einen Längsschnitt durch eine erste Ausführungsform der erfindungsgemäßen Kathetervorrichtung mit Port,
- Fig. 2: einen gegenüber Fig. 1 um 90° versetzten Längsschnitt der ersten Ausführungsform mit einer am proximalen Ende eingesetzten Nadel,
- Fig. 3: eine Kathetervorrichtung in der Schnittdarstellung mit einem Ventilöffnungselement,
- Fig. 4: ein in das proximale Ende des Katheteransatzes gemäß Figur 3 eingeführtes Anschlusselement,
- Fig. 5a - 5e: perspektivische Ansichten von Ausführungsformen des Schlauchelementes,
- Fig. 6: einen Längsschnitt einer erfindungsgemäßen Kathetervorrichtung mit dem in Fig. 5b wiedergegebenen Schlauchelement,
- Fig. 7: eine Kathetervorrichtung mit Nadel und Nadelschutzelement.
- Fig. 8a und 8b: ein Ventilbetätigungselement, und
- Fig. 9 - 12: weitere Kathetervorrichtungen.

In den Figuren ist mit 1 ein hohler Katheteransatz bezeichnet, dessen distales Ende mit einem Katheter 2 verbunden bzw. verbindbar ist. Am proximalen Ende ist der Katheteransatz 1 mit einer Verbindungseinrichtung, beispielsweise einem Luer-Nocken 3 für den Anschluss eines Infusionsschlauches oder dergleichen versehen. Zwischen proximalem und distalem Ende ist am Katheteransatz 1 seitlich ein Port 4 angebracht, mit dem beispielsweise eine Spritze verbunden werden kann, um eine Infusionsflüssigkeit durch den Katheter 2 dem Patienten zuzuführen. Im Bereich der Öffnung 4a des Ports ist im Katheteransatz 1 ein schlauchförmiges, wenigstens teilweise flexibles bzw. elastisches Element 5 angeordnet, das als ein erstes Ventilelement die Portöffnung 4a geschlossen hält und bei Anliegen von Druck an der Portöffnung 4a sich elastisch so verformt, dass eine Verbindung zwischen Portöffnung 4a und Katheter 2 freigegeben wird. Wenn kein Druck mehr an der Portöffnung anliegt, kehrt das Schlauchelement 5 aufgrund seiner Elastizität wieder in die dargestellte Schließstellung zurück.

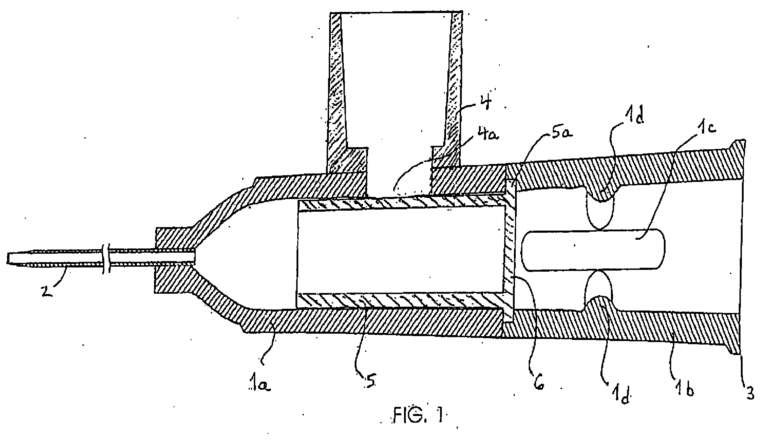

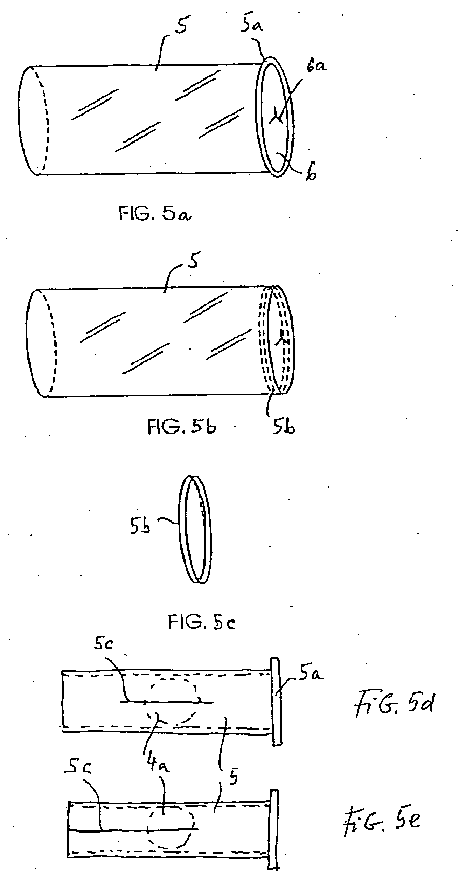

Das wenigstens teilweise flexible Schlauchelement 5 ist bei dem in Fig. 1 wiedergegebenen Ausführungsbeispiel am proximalen Ende durch eine Ventilscheibe 6 verschlossen, die mit wenigstens einem radial verlaufenden Schlitz 6a versehen ist. Fig. 5a zeigt drei sternförmig angeordnete Schlitze 6a. Zweckmäßigerweise besteht die ein zweites Ventil bildende Ventilscheibe 6 aus dem gleichen Material wie das Schlauchelement 5, es kann aber auch ein gesondertes Ventilelement in das proximale Ende oder im proximalen Bereich des Schlauchelementes 5 vor der Portöffnung 4a eingesetzt werden.

Die Ventilscheibe 6 ist als Zwei-Wege-Ventil ausgebildet, sodass eine Durchströmung von der proximalen zur distalen Seite und umgekehrt möglich ist. Damit die Ventilscheibe 6 nicht öffnet, wenn durch den Port 4 Injektionsflüssigkeit eingeführt wird, kann die Ventilscheibe 6 entsprechend steif ausgebildet werden, sodass sie nur für eine Durchströmung in proximale Richtung öffnet, wenn auf der proximalen Seite ein entsprechend starker Saugdruck anliegt, der höher ist als ein am Port 4 angelegter Druck. In einer nicht erfindungsgemäßen Variante wird ein Ventilbetätigungselement in Verbindung mit der Ventilscheibe 6 vorgesehen, wie dies nachfolgend näher erläutert wird.

Bei dem Ausführungsbeispiel nach Fig. 1 ist am Schlauchelement 5 am proximalen Ende ein radial abstehender Flansch 5a ausgebildet, der zwischen zwei Gehäuseteilen 1a und 1b in einer Nut in Achsrichtung gehalten ist. Durch diesen längs seines Umfangs zwischen den Gehäuseteilen 1a und 1b eingespannten Flansch 5a des Schlauchelementes 5 und die das Schlauchelement am proximalen Ende verschließende und versteifende Ventilscheibe 6 kann sich das Schlauchelement 5 am proximalen Ende nicht radial verformen, wenn an der Portöffnung 4a ein Druck anliegt, sodass das Schlauchelement 5 nur in dem Bereich zwischen distalem Ende und Portöffnung 4a so radial eingedrückt wird, dass eine Verbindung zwischen Portöffnung 4a und Katheter 2 freigegeben wird, während eine Verbindung zwischen Portöffnung 4a und proximalem Ende des Katheteransatzes 1 verschlossen bleibt. Dadurch, dass die Ventilscheibe 6 das proximale Ende des Schlauchelementes 5 abdeckt, kann auch keine Rückströmung der durch den Port 4 eingeführten Infusionsflüssigkeit zum proximalen Ende des Katheteransatzes auftreten.

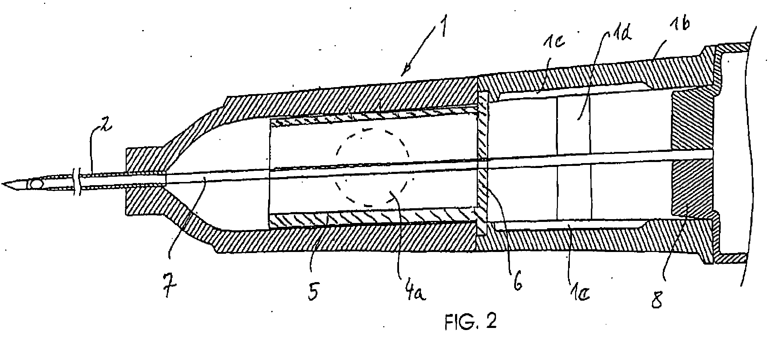

Fig. 2 zeigt eine in die Kathetervorrichtung eingesetzte Hohlnadel 7 an einem Nadelansatz 8, der in das proximale Ende des Katheteransatzes 1 eingesetzt ist, wobei sich die Nadel 7 unter Aufweitung der Schlitze 6a durch die Ventilscheibe 6 und den Katheter 2 erstreckt und mit der Spitze aus dem distalen Ende des Katheters vorsteht.

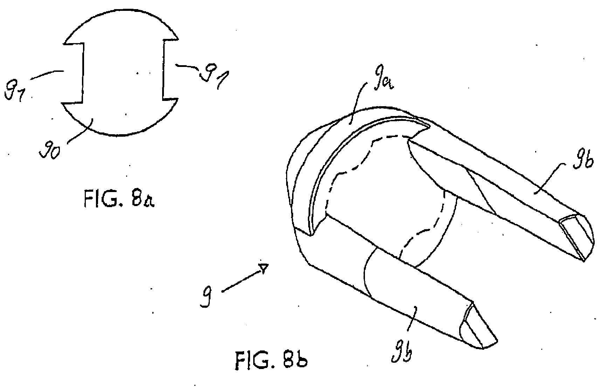

In einer nicht erfindungsgemäßen variante ist im Gehäuseteil 1b auf der proximalen Seite der Ventilscheibe 6 an diametral gegenüberliegenden Stellen eine Längsnut 1c ausgebildet, die zur Führung eines Ventilbetätigungselementes 9 in Achsrichtung des Katheteransatzes 1 dient. Fig. 8b zeigt ein Ausführungsbeispiel eines solchen Ventilbetätigungselementes 9, das auf diametral gegenüberliegenden Seiten eines Kopfteiles 9a zwei Führungsschenkel 9b aufweist, die in Achsrichtung von dem etwa kegelförmigen Kopfteil 9a abstehen und in die Führungsnuten 1c eingreifen.

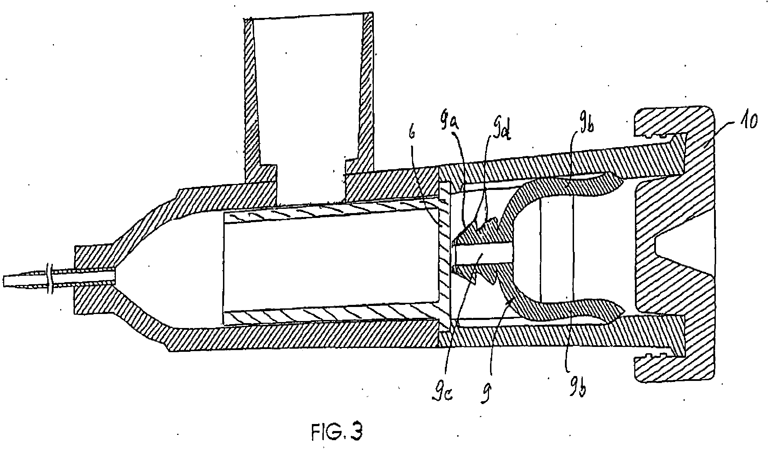

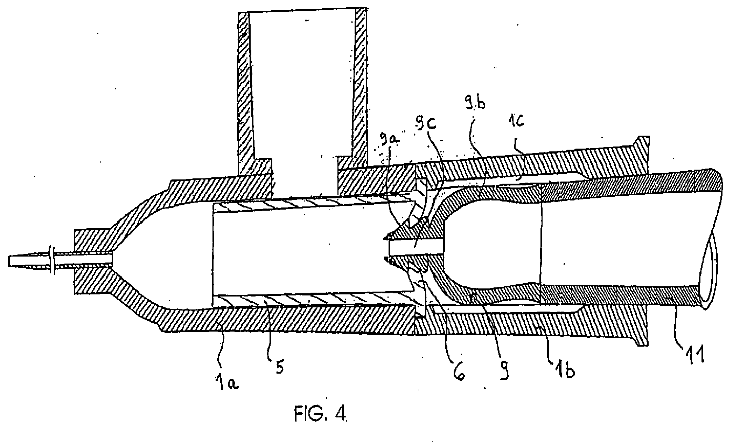

In den Fig. 3 und 4 ist das mit einer Durchgangsbohrung 9c versehene Kopfteil 9a mit einer Abstufung 9d versehen, damit das Ventilbetätigungselement 9 in der Ventilscheibe 6 nach Auseinanderdrücken der radialen Schlitze 6a einrasten kann, wie dies Fig. 4 zeigt. Hierbei ist ein rohrförmiges Anschlusselement 11 in das proximale Ende des Katheteransatzes 1 eingeführt, dessen Stirnseite an den Enden der Führungsschenkel 9b zum Anliegen kommt und diese in distale Richtung so verschiebt, dass das Kopfteil 9a des Ventilbetätigungselementes 9 die Ventilscheibe 6 öffnet.

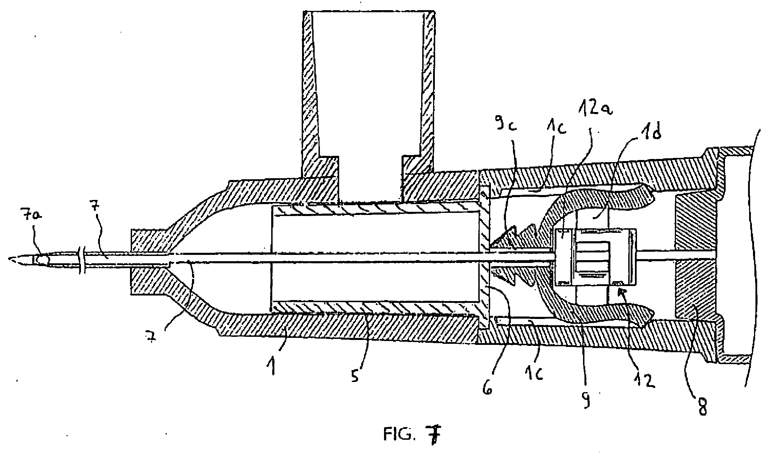

Fig. 7 zeigt die in die Kathetervorrichtung eingesetzte Hohlnadel 7, die sich durch die Bohrung 9c im Ventilbetätigungselement 9 und die Ventilscheibe 6 erstreckt, wobei zwischen Nadelansatz 8 und Ventilbetätigungselement 9 ein Nadelschutzelement 12 auf der Nadel 7 angeordnet ist, das im Katheteransatz 1 in der in Fig. 7 wiedergegebenen Bereitstellüng an einem radialen Vorsprung 1d gehalten wird, der sich bei dem dargestellten Beispiel in Umfangsrichtung zwischen den Führungsnuten 1c auf dem Innenumfang des Gehäuseteils 1b des Katheteransatzes erstreckt. Fig. 1 zeigt die beiderseits der Führungsnut 1c ausgebildeten, wulstförmigen Abschnitte des radialen Vorsprungs 1d teilweise im Schnitt. Wenn die Nadel 7 aus der in Fig. 7 wiedergegebenen Bereitstellung des Nadelschutzelementes 12 aus dem Katheteransatz zurückgezogen wird, wird das Nadelschutzelement 12 im Katheteransatz 1 zunächst durch den radialen Vorsprung 1d gehalten, bis eine nahe der Nadelspitze vorgesehene Eingriffseinrichtung 7a vorzugsweise in der Form einer Nadelquetschung, die zu diametral gegenüberliegenden Vorsprüngen am Nadelumfang führt, mit der proximalen Rückwand des Nadelschutzelementes 12 in Eingriff tritt und bei weiterem Zurückziehen der Nadel 7 das Nadelschutzelement 12 mitnimmt, wobei die distalen Enden der Arme 12a die Nadelspitze übergreifen und blockieren, indem sie sich radial nach innen bewegen und sich dabei von dem radialen Vorsprung 1d lösen, sodass das Nadelschutzelement 12 zusammen mit der Nadel aus der Kathetervorrichtung herausgezogen werden kann.

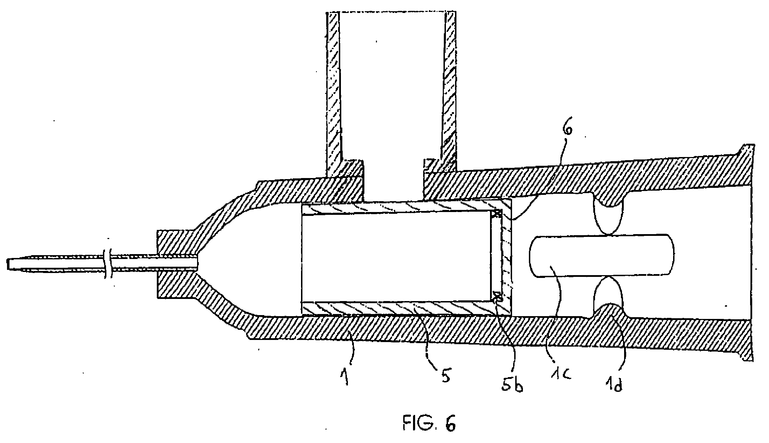

Das Schlauchelement 5 kann am proximalen Ende oder im proximalen Bereich auch durch einen innen liegenden Ring 5b versteift ausgebildet werden, wie dies Fig. 5b und Fig. 5c zeigen. Fig. 6 zeigt einen Längsschnitt durch eine Kathetervorrichtung mit einem am proximalen Ende derart versteiften Schlauchelement 5. Bei dieser Ausführungsform des Schlauchelementes kann der Katheteransatz 1 auch einstückig ausgebildet sein, wie Fig. 6 zeigt. Der nicht flexible Versteifungsring 5b kann auch in einem Abstand vom proximalen Ende des Schlauchelementes in dieses eingesetzt sein, wobei er zwischen proximalem Ende des Schlauchelementes und Portöffnung 4a angeordnet wird..

Ein Versteifungsring 5b oder ein entsprechendes Versteifungselement kann auch in Verbindung mit einem Flansch 5a vorgesehen werden.

Fig. 5d zeigt eine Ausführungsform des Schlauchelementes 5, das einen Schlitz 5c in Achsrichtung im Bereich der Portöffnung 4a aufweist, um bei Einführen von Injektionsflüssigkeit durch den Port 4 ein Öffnen des Schlauchelementes 5 zu erleichtern. Bei der in Fig. 5e wiedergegebenen Ausführungsform erstreckt sich der Schlitz vom Bereich der Portöffnung 4a bis zum distalen Ende des Schlauchelementes 5, während der Schlitz bei der Ausführungsform nach Fig. 5d nur im Bereich der Portöffnung ausgebildet ist, sodass beim Einführen von Injektionsflüssigkeit durch den Port 4 sich nur die Ränder des Schlitzes verformen und der distale Abschnitt des Schlauchelementes am Innenumfang des Katheteransatzes anliegend bleiben kann.

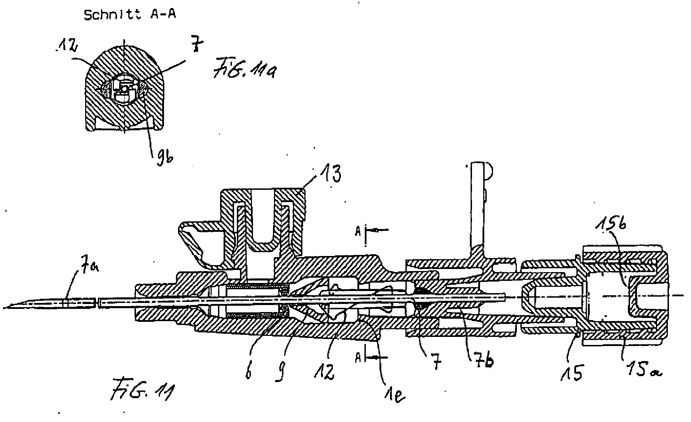

Das proximale Ende des Katheteransatzes 1 kann durch eine Verschlusskappe 15a (Fig. 11) verschlossen gehalten werden, wenn über den Port 4 eine Injektion ausgeführt wird.

Anstelle der beschriebenen Ausführungsform eines flexiblen Schlauchelementes 5, das sowohl ein erstes Ventilelement an der Portöffnung 4a als auch ein zweites Ventilelement im proximalen Bereich des Katheteransatzes 1 bildet, können auch für die Portöffnung und die Katheteransatzöffnung jeweils getrennte Ventilelemente vorgesehen sein.

Anstelle eines Ports 4 kann auch eine Kathetervorrichtung mit zwei Ports am Katheteransatz vorgesehen sein, wobei das erste Ventil beide Portöffnungen verschließt und freigibt. Es können dabei auch zwei getrennte Ventilelemente für die beiden Portöffnungen vorgesehen werden.

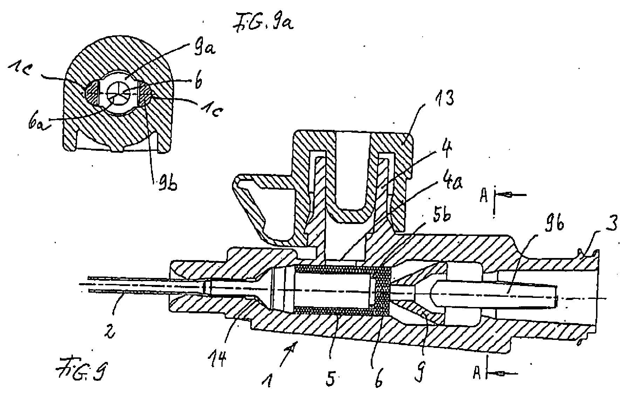

Fig. 9 - 12 zeigen Kathetervorrichtungen, die teilweise denen der Fig. 1 - entspreche, wobei für gleiche oder entsprechende Bauteile die gleichen Bezugszeichen verwendet sind.

Fig. 9 zeigt einen einteiligen Katheteransatz 1, bei dem der Port 4 durch eine Kappe 13 verschlossen ist. Der Katheter 2 ist durch ein trichterförmiges Befestigungselement 14 im Katheteransatz 1 durch Klemmen befestigt. Die Wandstärke der Ventilscheibe 6 ist verstärkt gegenüber der Wandstärke am Schlauchelement 5 dargestellt. Hierdurch kann das Schlauchelement 5 bei einem an der Portöffnung 4a anliegenden Druck durch Verformen die Portöffnung 4a öffnen, während die Ventilscheibe 6 geschlossen bleibt. Bei dem dargestellten Beispiel in Fig. 9 ist zusätzlich ein Ventilbetätigungselement 9 vorgesehen, das die Ventilscheibe 6 auf der proximalen Seite abstützt, wenn auf der distalen Seite ein Druck anliegt, sodass die Ventilscheibe in proximale Richtung nicht öffnet, wenn am Port 4 ein Druck anliegt.

Fig. 9a zeigt einen Schnitt längs der Linie A-A in Fig. 9, wobei die sternförmig angeordneten Schlitze 6a der Ventilscheibe 6 durch die Durchgangsöffnung 9c des Ventilbetätigungselementes 9 sichtbar ist. Die in den in Achsrichtung verlaufenden Führungsnuten 1c eingreifenden Führungsschenkel 9b sind an einem trichterförmigen Ventilbetätigungselement 9 angeformt.

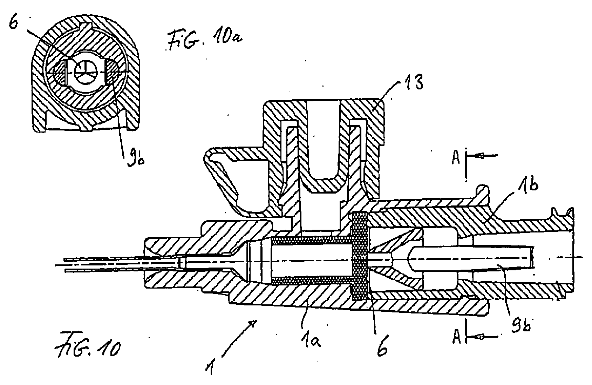

Fig. 10 zeigt in entsprechender Darstellung wie Fig. 9 einen zweiteiligen Katheteransatz 1, bei dem die Ventilscheibe 6 in radialer Richtung verbreitert ausgebildet und durch das Gehäuseteil 1b im Gehäuseteil 1a des Katheteransatzes gehalten ist.

Bei diesem Beispiel ist, wie die Schnittansicht in Fig. 10a zeigt, das Gehäuseteil 1b nicht rotationssymmetrisch ausgebildet, sondern im oberen Bereich verstärkt und mit einer in Achsrichtung verlaufenden Rippe auf dem Außenumfang versehen, der in eine entsprechende Längsnut auf dem Innenumfang des Gehäuseteils 1a eingreift. Diese Ausgestaltung verhindert ein Verdrehen zwischen den beiden Gehäuseteilen, wenn z. B. das Anschlusselement 16 angeschraubt wird.

Fig. 11 zeigt eine an einem Nadelansatz 7b befestigte Nadel 7, die sich durch das Ventilbetätigungselement 9, die Ventilscheibe 6 und den Katheter 2 erstreckt, wobei auf der Nadel ein Nadelschutzelement 12 mit sich kreuzenden Armen zwischen den beiden Führungsschenkeln 9b des Ventilbetätigungselementes 9 angeordnet ist. Das Nadetschutzelement 12 in Fig. 11 entspricht dem in Fig. 7 in einer um 90° verdrehten Ansicht. Bei den Beispiel nach Fig. 11 ist auch der Nadelansatz 7b durch eine Kappe 15 verschlossen. Am proximalen Ende der Kathetervorrichtung ist eine abnehmbare Verschlusskappe 15a aufgesteckt. Nach Entfernen der Nadel 7 mit Nadelschutzelement kann die Verschlusskappe 15a auf das offene proximale Ende des Katheteransatzes aufgeschraubt werden. Vorzugsweise ist die Verschlusskappe 15a mit einem verkürzten Konus 15b versehen, damit das Ventilbetätigungselement 9 durch die Verschlusskappe nicht beaufschlagt wird, wie dies auch in Fig. 3 der Fall ist.

Bei dem Beispiel nach Fig. 11 wird das Nadelschutzelement 12 im Katheteransatz 1 gehalten, wenn die Nadel 7 aus dem Katheteransatz zurückgezogen wird, indem die gegenüberliegenden Arme an einem Absatz 1e zum Anliegen kommen. Sobald die Nadelspitze zwischen die beiden Arme des Nadelschutzelementes 12 zu liegen kommt, schwenken die beiden Arme nach innen, sodass die Nadel mit dem Nadelschutzelement 12 an der Nadelspitze aus dem Katheteransatz 1 herausgezogen werden kann.

Auch kann das Nadelschutzelement 12 mit dem Ventilbetätigungselement 9 bzw. mit dessen Führungsschenkeln 9b so in Eingriff stehen, dass das Nadelschutzelement 12 durch das Ventilbetätigungselement 9 gehalten wird, bis das Nadelschutzelement durch Aufnahme der Nadelspitze in die Schutzstellung gelangt, worauf die Klemmverbindung mit dem Ventilbetätigungselement 9 gelöst wird und das Nadelschutzelement 12 mit der Nadel 7 aus dem Katheteransatz 1 herausgezogen werden kann.

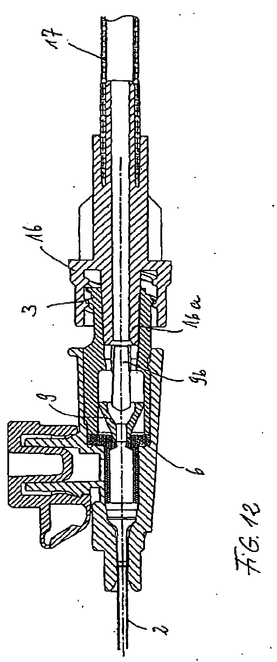

Fig. 12 zeigt ein Anschlusselement 16, das bei der Kathetervorrichtung nach Fig. 10 auf dem Luer-Nocken 3 aufgeschraubt ist, wobei der in distale Richtung vorspringende, rohrförmige Ansatz 16a das Ventilbetätigungselement 9 über die Führungsschenkel 9b so beaufschlagt, dass die Ventilscheibe 6 geöffnet ist. In dieser in Fig. 12 wiedergegebenen Stellung kann über eine an das Anschlussstück 16 angeschlossene Leitung 17 Blut entnommen werden.

In Fig. 11 wird das Ventilbetätigungselement 9 durch den Nadelansatz 7b nicht beaufschlagt, sodass das Ventilbetätitungselement 9 in der in Fig. 9 wiedergegebenen Stellung verbleibt. Lediglich die Nadel 7 wird durch die Ventilscheibe 6 geführt, wobei die zwischen den Ventilschlitzen 6a liegenden elastischen Bereiche der z. B. aus Silicon bestehenden Ventilscheibe 6 auf dem Außenumfang der Nadel 7 anliegen und Blut von der Nadel abstreifen, wenn die Nadel 7 aus dem Katheteransatz 1 zurückgezogen wird.

Das Ventilbetätigungselement 9 kann auch mit einer Abstreifeinrichtung verschen sein, wie in DE 20 2006 017 732 beschrieben, beispielsweise in Form eines die Nadel umgebenden Abstreifrings in dem Durchlass 9c (Fig. 3) des Ventilbetätigungselementes 9.

Fig. 8a zeigt einen Abstreifer 90 aus einem flüssigkeitsundurchlässigen Film, wie z.B. einen Polyethylenfilm, der eine im Wesentlichen kreisförmige Gestalt mit zwei Ausschnitten 91 umfasst. Die Ausschnitte 91 sind so dimensioniert und geformt, dass sie an dem Ventilbetätigungselement 9 angebracht werden können und die Führungsschenkel 9b aufnehmen. In einem Beispiel weist der Abstreifer 90 einen durchgehend mittigen Abschnitt (d.h., keine mittige Öffnung) auf, die zum Durchbohren durch eine Nadel während des Zusammenbaus ausgebildet ist. Wenn die Nadel 7 aus dem Katheteransatz 1 zurückgezogen wird, wird Blut vom Nadelumfang durch den Abstreifer 90 abgestreift.

Bei einer solchen Ausgestaltung mit einem Abstreifer 90 kann bei Blutentnahme auf der proximalen Seite des Katheteransatzes das Blut um das Ventilbetätigungselement mit Abstreifer fließen, wobei Blut auch durch das Loch im Abstreifer 90 austreten kann, das durch die Nadel eingestochen wurde. Es können auch Nuten in Achsrichtung auf dem kegelstumpfförmigen Kopfteil 9a ausgebildet sein, um die Blutentnahme zu begünstigen.

Fig. 8b zeigt eine perspektivische Ansicht eines Ventilbetätigungselementes 9 mit einem kegelstumpfförmigen Kopfteil 9a und zwei Führungsschenkeln 9b, die mit dem Kopfteil verbunden sind und sich proximal von diesem erstrecken. Der Durchlass am Kopfteil weist eine trichterförmige Gestalt auf. In der Darstellung erstrecken sich die zwei Schenkel 9b parallel und versetzt in Bezug auf eine Achse, die durch die Mitte des Durchlasses bestimmt wird; sie können jedoch auch radial nach außen abgewinkelt sein, wobei sie sich proximal erstrecken. Die zwei Schenkel können auch einen leichten Bogen oder eine leichte Krümmung aufweisen.

Fig. 7 zeigt das in Fig. 11 in einer Seitenansicht wiedergegebene Nadelschutzelement 12 mit sich kreuzenden Armen in einer Draufsicht Anstelle eines solchen Nadelschutzelementes 12 mit sich kreuzenden Armen kann auch eine andere Ausführungsform eines Nadelschutzelements vorgesehen werden, durch das beim Zurückziehen der Nadel die Nadelspitze abgedeckt und blockiert wird, sodass keine Verletzung durch die Nadelspitze möglich ist.

## Patentansprüche

1. Kathetervorrichtung, umfassend
einen hohlen Katheteransatz (1), dessen distales Ende mit einem Katheter (2) verbindbar ist und an dessen proximalem Ende eine Verbindungseinrichtung (3) für eine zusätzliche Einrichtung vorgesehen ist,
einen zwischen proximalem und distalem Ende radial abzweigenden Port (4) am Katheteransatz (1),
ein erstes Ventilelement (5) in dem Katheteransatz (1), wobei das erste Ventilelement (5) in Form eines flexiblen Schlauchelementes ausgebildet ist, das in der Bereitstellung auf dem Innenumfang des Katheteransatzes (1) anliegt und den Port (4) verschließt und bei Anliegen von Druck von außen am Port die Verbindung zwischen Port und Katheter durch Deformation freigibt, und
ein zweites Ventilelement (6), das das Austreten von Blut aus dem Katheteransatz in proximale Richtung verhindert, wobei das zweite Ventilelement (6) durch eine das proximale Ende des ersten Ventilelements (5) verschließende Ventilscheibe (6) mit
wenigstens einem Schlitz ausgebildet ist,
**dadurch gekennzeichnet,**
**dass** die Ventilscheibe (6) als Zwei-Wege-Ventil derart ausgebildet ist, dass eine Durchströmung in beiden Richtungen möglich ist, und wobei die Ventilscheibe (6) nur für eine Durchströmung in proximale Richtung bei Anliegen eines entsprechend starken Saugdrucks auf der proximalen Seite der Ventilscheibe (6) öffnet.

2. Kathetervorrichtung nach Anspruch 1, wobei die Ventilscheibe (6) mit wenigstens einem radial verlaufenden Schlitz (6a) versehen ist.

3. Kathetervorrichtung nach Anspruch 1 oder 2, wobei am proximalen Ende des Schlauchelementes (5) ein radial abstehender Flansch (5a) ausgebildet ist, der in eine Ringnut auf dem Innenumfang des Katheteransatzes (1) eingreift.

4. Kathetervorrichtung nach Anspruch 1 bis 3, wobei am proximalen Ende des Schlauchelementes (5) auf dessen Innenumfang ein Versteifungsring (5b) eingesetzt ist.

5. Kathetervorrichtung nach Anspruch 4, wobei der Katheteransatz (1) einstückig ausgebindet ist.

6. Kathetervorrichtung nach einem der Ansprüche 1 bis 4, wobei der Katheteransatz (1) aus zwei Gehäuseteilen (1a) und (1b) ausgebildet ist.

7. Kathetervorrichtung nach einem der vorhergehenden Ansprüche, wobei auf der proximalen Seite des zweiten Ventilelementes (6) ein Nadelschutzelement (12) auf einer in die Kathetervorrichtung eingesetzten Nadel (7) verschiebbar geführt ist.

8. Kathetervorrichtung nach Anspruch 7, wobei auf dem Innenumfang des Katheteransatzes (1) eine Halteeinrichtung (1d) für das Nadelschutzelement (12) ausgebildet ist.

9. Kathetervorrichtung nach einem der Ansprüche 1 bis 8, wobei das erste Ventilelement bildende Schlauchelement (5) im Bereich der Portöffnung (4a) mit einem in Achsrichtung verlaufenden Schlitz (5c) versehen ist.

## Claims

1. A catheter assembly, comprising
a hallow catheter hub (1), the distal end of which is connectable to a catheter (2) and on the proximal end of which connection means (3) for an additional means are provided,
a port (4) on the catheter hub (1) radially branching off between the proximal and the distal ends,
a first valve element (5) in the catheter hub (1), wherein the first valve element (5) is formed in the form of a flexible hose member, which abuts on the inner circumference of the catheter hub (1) and closes the port (4) in the ready positon, and unblocks the connection between the port and the catheter by deformation, when external pressure is applied to the port, and
a second valve element (6) that prevents emergence of blood from the catheter hub in the proximal direction, wherein the second valve element (6) is formed by a valve disc (6) provided with at least one slit , which closes the proximal end of the first valve element (5),
**characterised in that**
the valve disc (6) is formed as a two-way valve such that throughflow in both directions is possible, and wherein the valve disc (6) opens for a throughflow in a proximal direction only if a correspondingly strong suction pressure is applied on the proximal side of the valve disc (6).

2. The catheter assembly according to claim 1, wherein the valve disc (6) is provided with at least one radially extending slit (6a).

3. The catheter assembly according to claim 1 or 2, wherein at the proximal end of the hose member (5), a radially projecting flange (5a) is formed that engages in an annular groove on the inner circumference of the catheter hub (1).

4. The catheter assembly according to claims 1 to 3, wherein at the proximal end of the hose member (5) on the inner circumference thereof, a reinforcing ring (5b) is inserted.

5. The catheter assembly according to claim 4, wherein the catheter hub (1) is formed in one piece.

6. The catheter assembly according to any one of claims 1 to 4, wherein the catheter hub (1) is formed by two hub parts (1a) and (1b).

7. The catheter assembly according to any one of the preceding claims, wherein on the proximal side of the second valve element (6), a needle protective element (12) is displaceably guided on a needle (7) inserted in the catheter assembly.

8. The catheter assembly according to claim 7, wherein on the inner circumference of the catheter hub (1), a holding means (1d) for the needle protective element (12) is formed.

9. The catheter assembly according to any one of claims 1 to 8, wherein the hose member (5) forming the first valve element in the area of the port opening (4a) is provided with a slit (5c) extending in the axial direction.

## Revendications

1. Ensemble de cathéter, comprenant
un embout de cathéter creux (1) dont l'extrémité distale peut être reliée à un cathéter (2) et à l'extrémité proximale duquel il est prévu un dispositif de connexion (3) pour un système supplémentaire,
un port bifurquant radialement entre l'extrémité proximale et l'extrémité distale (4) au niveau de l'embout de cathéter (1),
un premier élément à soupape (5) dans l'embout de cathéter (1), le premier élément à soupape (5) étant réalisé sous forme d'un élément tubulaire souple qui, en position prête à l'emploi, repose sur la circonférence interne de l'embout de cathéter et ferme le port (4) et qui, en cas d'application de pression depuis l'extérieur sur le port, autorise la connexion entre le port et le cathéter par déformation et
un second élément à soupape (6) qui empêche la sortie de sang de l'embout de cathéter dans le sens proximal, le second élément à soupape (6) étant constitué par un disque de soupape (6) fermant l'extrémité proximale du premier élément à soupape (5) et comportant au moins une fente,
**caractérisé en ce que**
le disque de soupape (6) est réalisé sous la forme d'une soupape à deux voies de manière à ce qu'un débit d'écoulement soit possible dans les deux sens, et dans lequel le disque de soupape (6) ne s'ouvre que pour un débit d'écoulement dans le sens proximal en cas d'application d'une pression d'aspiration de force adéquate sur la face proximale du disque de soupape (6).

2. Ensemble de cathéter selon la revendication 1, dans lequel le disque de soupape (6) est pourvu d'au moins une fente orientée radialement (6a).

3. Ensemble de cathéter selon la revendication 1 ou 2, dans lequel, à l'extrémité proximale de l'élément tubulaire (5), est réalisée une bride dépassant radialement (5a) qui s'engrène dans une rainure annulaire sur la circonférence interne de l'embout de cathéter (1).

4. Ensemble de cathéter selon la revendication 1 à 3, dans lequel, à l'extrémité proximale de l'élément tubulaire (5), sur sa circonférence interne, une bague de renfort (5b) est insérée.

5. Ensemble de cathéter selon la revendication 4, dans lequel l'embout de cathéter (1) a une conformation monobloc.

6. Ensemble de cathéter selon une des revendications 1 à 4, dans lequel l'embout de cathéter (1) est composé de deux pièces de boîtier (1a) et (1b).

7. Ensemble de cathéter selon une des revendications précédentes, dans lequel, sur la face proximale du second élément à soupape (6), un élément protecteur d'aiguilles (12) est guidé de manière déplaçable sur une aiguille (7) insérée dans l'ensemble de cathéter.

8. Ensemble de cathéter selon la revendication 7, dans lequel, sur la circonférence interne de l'embout de cathéter (1), un élément de retenue (1d) pour l'élément protecteur d'aiguille (12) est constitué.

9. Ensemble de cathéter selon une des revendications 1 à 8, dans lequel l'élément tubulaire formant le premier élément à soupape (5) est pourvu, au niveau de l'ouverture du port (4a), d'une fente (5c) orientée dans le sens de l'axe.
